# EUROPEAN PATENT APPLICATION

(11) **EP 0 752 402 A1**
(43) Date of publication of application: **08.01.1997**
(21) Application number: 96109098.2
(22) Date of filing: 06.06.1996
(51) Int. Cl.: C07C 5/333, C07C 15/46

(54) **Process for dehydrogenation of ethylbenzene to styrene**

(30) Priority: 07.06.1995 US 472590
(71) Applicant: FINA TECHNOLOGY, INC., Dallas, Texas 75206 (US)
(72) Inventor: Butler, James R., Houston, Texas 77059 (US); Merrill, James T., Katy, Texas 77449 (US)
(74) Representative: Leyder, Francis

(57) **Abstract**

A method of dehydrogenating ethylbenzene to styrene and aromatics, said method comprising passing ethylbenzene over dehydrogenation catalyst beds in a plurality of serially-arranged dehydro reactors, wherein at least the first of said reactors contains a high-selectivity dehydro catalyst, and at least the last of said reactors contains a low-selectivity dehydro catalyst.

## Description

### BACKGROUND OF THE INVENTION

This invention relates to the field of styrene manufacture and more particularly discloses methods and apparatus, including reactor vessels, for the dehydrogenation of ethylbenzene into styrene monomer.

It is well known in the art of styrene manufacture to react ethylbenzene ("EB") over a dehydrogenation catalyst such as iron oxide under elevated temperatures in the range of 1000-1200°F and at a pressure of about 10 to 20 PSIA in order to strip hydrogen from the ethyl-radical on the benzene ring to form the styrene molecule. This might normally be done in a series of styrene radial reactors which are commonly termed EB dehydro reactors. The dehydro reactors generally are elongated cylindrical vertical structures of a very large size ranging in diameter from about five to about thirty feet or more and in length from about ten to about one hundred feet or more. The normal construction for such a reactor allows for input of the ethylbenzene gas at an inlet located in the bottom center of the vertical reactor, whereupon the gas is flowed up through an annular area, passing radially outward through a porous catalyst bed of iron oxide or other suitable dehydro catalyst, and then passing upward through an outer annular area to exit at the top of the reactor shell. Since the flow of ethylbenzene across the catalyst bed is in a radial direction, these reactors are sometimes identified as "radial" reactors.

It is currently believed by those skilled in the art of styrene manufacture that the optimum arrangement of multiple, radial bed, EB reactors with iron oxide dehydro catalyst beds is to place in the first one or two reactors of the series a low-selectivity dehydro catalyst, and to locate in the downstream reactors the dehydro catalyst having high selectivity. "Selectivity" in this instance is considered by one skilled in the art to mean the ability of the catalyst to selectively produce higher levels of the desirable styrene and lower levels of the undesirable toluene and benzene. "Activity" is considered to be the ability of the catalyst to convert a certain percentage of ethylbenzene to aromatics for each pass of feedstock over the catalyst.

Radial reactors particularly useful in manufacturing styrene from ethylbenzene are those types disclosed in U.S. Patent 5,358,698 to Butler et al., issued October 25, 1994; which patent is hereby incorporated by reference herein.

It has been found, however, that the normal arrangement of placing the low-selectivity catalyst upstream of the high-selectivity catalyst is not a desirable arrangement when using multiple radial bed reactors and an optimum iron oxide catalyst When such an arrangement is attempted, the result is loss of activity and productivity, leading one to believe that multiple reactors cannot be utilized with varied-selectivity catalysts.

### SUMMARY OF THE INVENTION

The present invention discloses a method of using multiple-bed radial reactors having varied-selectivity catalysts to dehydrogenate EB into styrene by using a high-selectivity iron oxide catalyst in the upstream reactors, followed by a low-selectivity catalyst in the downstream reactor or reactors.

### BRIEF DESCRIPTION OF THE DRAWING

The figure is a cross sectional schematic representation of multiple, vertically displaced, radial bed reactor vessels comprising the reactor system for practicing the present invention.

### DESCRIPTION OF THE PREFERRED EMBODIMENTS

Referring now to the drawing, a typical multiple bed reactor system is indicated comprising typically three vertical radial bed dehydro reactors 10, 110 and 210. Each EB dehydro reactor vessel has an elongated outer cylindrical shell 11 enclosing an inner cylindrical displacement member 12 located concentrically inside cylindrical shell 11. Shell 11 and displacement member 12 are generally cylindrical, meaning that a cross sectional view taken perpendicular to the longitudinal center lines of these two vessels would be circular in shape. Preferably shell 11 is a circular right-cylinder and displacement member 12 is non-circular cylinder such as a conical or frustoconical shape. The displacement member 12 is located coaxially within shell 11, meaning that preferably the central longitudinal axes of each of these two structures coincide with each other.

An inlet pipe 13, having a relatively large cross-sectional area, is connected to a central inlet opening 14 formed in the bottom of shell 11. Preferably inlet pipe 13 is also cylindrical in cross sectional area and enters the vessel after making a 90° turn from horizontal. Inlet pipe 13 has a series of flow baffles 16, attached internally to the walls thereof to control the flow of gas therethrough and offset the affect of the right angle turn in pipe 13.

The placement of cylinder 12 within shell 11 in a coaxial alignment serves to form an annular catalyst area 18 therebetween. A cylindrical annular catalyst bed 19 is located between displacement member 12 and shell 11. A series of radially outwardly extending flow baffles 20 are formed on the outer wall of catalyst bed 19, extending radially outward therefrom to further direct flow of gases through the catalyst bed and into a radial direction, thereby preventing longitudinal flow and further smoothing out flow across the catalyst bed.

Catalyst bed 19 comprising a concentric cylindrical catalyst shell made of a perforated or porous inner wall and a similar porous or perforated outer wall. Preferably the catalyst shell is sufficient to maximize flow and still retain the dehydro catalyst between the inner and outer walls. The typical catalyst utilized in catalyst bed 19 is an iron oxide catalyst of variable selectivity, more particularly described hereinbelow.

The sizing of the flow areas of inlet pipe 13 and the annular area 21 between the displacement member 12 and the catalyst bed 19 is preferably in the range of about 2:1 with annular area 21 being approximately twice the value of the cross sectional area of inlet pipe 13. Furthermore, the annular area 22 between catalyst bed 19 and shell 11 is approximately 5 to 6 times the annular flow area 21.

Each of the three vertical EB dehydro reactors 10, 110, and 210, are constructed similarly and a description relating to reactor vessel 10 also applies to the second and third reactors.

Reactor 10 has an outlet opening 25 communicating with outlet pipe 26, which flows into the inlet pipe 113 of reactor 110. Likewise, reactor 110 has an outlet opening 125 communicating with a flow pipe 126, which in turn is connected to inlet 213 of the third reactor 210. Reactor 210 has an outlet opening 225 communicating with a product stream flow conduit 226 containing the finished styrene monomer.

According to the present invention, the catalyst bed 19 located in reactor 10 and the catalyst bed 19 in reactor 110 are preferably of a high selectivity iron oxide catalyst such as that sold by Criterion Catalyst Inc. of Houston, Texas and designated as 025HA. Conversely, the catalyst bed 19 in reactor 210, is a low selectivity iron oxide catalyst such as that sold by Criterion Catalyst Inc. and designated as 025HB. As mentioned previously, conventional technology would indicate that the low selectivity catalyst should be utilized in the first and/or second reactors and the high selectivity catalyst then located in the remaining downstream reactors. It was discovered however, that the opposite was in fact that case and that placing the low selectivity catalyst in the primary reactor when using the optimum iron oxide EB dehydro catalyst resulted in a loss of activity and production. Therefore, the high selectivity catalyst comprising the previously mentioned Criterion iron oxide catalyst was placed in the upstream reactors 10 and 110 and the previously described Criterion low selectivity iron oxide catalyst was placed in the remaining downstream reactor 210. As a result of this unconventional catalyst arrangement, the ethylbenzene dehydrogenation system of the figure was found to operate very efficiently with a high selectivity and high activity result and a long-term activity period with no loss of production. Thus, is a disclosed a system for utilizing multiple radial bed vertical reactors utilizing highly optimized iron oxide catalyst for the dehydrogenation of EB by placing the high selectivity catalyst upstream or ahead of the low selectivity catalyst as opposed to the conventional belief that the low selectivity catalyst must proceed the high selectivity catalyst.

In typical operation therefore, EB feedstock is supplied to the upstream reactor vessel 10 via feed supply line 13 through the lower inlet opening of vessel 10. From there the EB feedstock flows into the annular area located radially inside the catalyst bed 19. The gas then flows radially outward through catalyst bed 19 where it is dehydrogenated to styrene and other aromatics. Operating conditions in each of the reactors are preferably in the range of about 900 to 1225°F and about 8-22 PISA pressure. Flow velocities in the reactor range from about 100 to about 400 feet per second, with a preferred overall flow velocity through the reactors of around 200 to 300 fps.

Although certain preferred embodiments of the present invention have been herein described in order to provide an understanding of the general principals of the invention, it will be appreciated that various changes and innovations can be affected in the described dehydrogenation reactor system without departing from these principals. For example, whereas the preferred embodiment utilizes a particular CRITERION iron oxide catalyst, it is believed that one skilled in the art could utilize other dehydro catalysts in the reverse order disclosed herein and obtain similar results. Other changes would be apparent to one skilled in the art and therefore the invention is declared to cover all changes and modifications of the specific examples of the invention herein disclosed for purposes of illustration, which do not constitute departure from the spirit and scope of the invention.

The embodiments of the present invention in which an exclusive property or privilege is claimed are defined as follows:

## Claims

1. A method of dehydrogenating ethylbenzene to styrene and aromatics, said method comprising passing ethylbenzene over dehydrogenation catalyst beds in a plurality of serially-arranged dehydro reactors, wherein at least the first of said reactors contains a high-selectivity dehydro catalyst, and at least the last of said reactors contains a low-selectivity dehydro catalyst.

2. The method of Claim 1 wherein said high selectivity catalyst is contained in the first two of said reactors and a third reactor contains said low selectivity catalyst.

3. The method of Claim 1 wherein said high selectivity catalyst is an iron oxide dehydro catalyst, and said low selectivity catalyst is an iron oxide dehydro catalyst exhibiting lower selectivity to styrene production than said high selectivity catalyst.

4. A method of dehydrogenating ethylbenzene to styrene and aromatics, said method comprising passing gaseous ethylbenzene over dehydrogenation catalyst beds in a plurality of serially-arranged, vertical, radial bed dehydro reactors, wherein at least the first of said reactors contains a high selectivity dehydro catalyst, and at least the last of said reactors contains a low selectivity dehydro catalyst.

5. The method of Claim 4 wherein said plurality of reactors comprises at least three reactors and said high selectivity dehydro catalyst is contained in at least the first two of said reactors, and said low selectivity catalyst is contained in at least the last of said reactors.

6. A method of dehydrogenating ethylbenzene to styrene, toluene and benzene, said method comprising passing gaseous ethylbenzene over iron oxide dehydrogenating catalyst beds in a plurality of serially-arranged, vertical, radial bed dehydro reactors, wherein at least the first of said reactors contains a high selectivity iron oxide catalyst and at least the last of said reactors contains a low selectivity iron oxide dehydro catalyst.

7. A method of Claim 6 wherein said plurality of reactors comprises at least three serially-arranged reactors.

8. The method of Claim 7 wherein said reactors operate at temperatures of around 900°F to 1200°F, pressures of about 8-22 PSIA and flow velocities of from 100 to 400 feet per second.
